# FASCICULE DE BREVET EUROPEEN

(11) **EP 1 570 050 B1**
(45) Date de publication et mention de la délivrance du brevet: **02.01.2008**
(21) Numéro de dépôt: 03813159.5
(22) Date de dépôt: 10.12.2003
(51) Int. Cl.: C12N 1/20, C12N 15/74, C12P 7/56

(54) **SOUCHES BACTERIENNES DU GENRE EXIGUOBACTERIUM, PROCEDE DE CULTURE ET APPLICATIONS**
BAKTERIELLE STÄMME VON EXIGUOBAKTERIUM, DEREN ZELLKULTUR UND VERWENDUNG
BACTERIAL STRAINS OF GENUS EXIGUOBACTERIUM, CULTURE METHOD AND USES

(30) Priorité: 13.12.2002 FR 0215865
(43) Date de publication de la demande: 07.09.2005
(73) Titulaire: Institut de Recherche pour le Développement ( IRD), 75480 Paris Cedex 10 (FR)
(72) Inventeur: FARDEAU, Marie-Laure, F-13170 LES PENNES-MIRABEAU (FR); COMBET-BLANC, Yannick, F-13009 MARSEILLE (FR); OLLIVIER, Bernard, F-13360 ROQUEVAIRE (FR)
(74) Mandataire: Peaucelle, Chantal
(86) Numéro de dépôt international: PCT/FR2003/003665
(87) Numéro de publication internationale: WO 2004/055173

(56) Documents cités:
- US-A- 6 022 537
- DRANCOURT MICHEL ET AL: "16S ribosomal DNA sequence analysis of a large collection of environmental and clinical unidentifiable bacterial isolates." JOURNAL OF CLINICAL MICROBIOLOGY, vol. 38, no. 10, octobre 2000 (2000-10), pages 3623-3630, XP002247318 ISSN: 0095-1137 -& DATABASE EMBL [en ligne] Bacterium str. 61610 16S ribosomal RNA gene, 3 mars 2000 (2000-03-03) retrieved from EBI Database accession no. AF227839 XP002247321
- FARROW JOHN A E ET AL: "Phylogenetic interrelationships of round-spore-forming bacilli containing cell walls based on lysine and the non-spore-forming genera Caryophanon, Exiguobacterium, Kurthia, and Planococcus." INTERNATIONAL JOURNAL OF SYSTEMATIC BACTERIOLOGY, vol. 44, no. 1, 1994, pages 74-82, XP009013848 ISSN: 0020-7713 -& DATABASE EMBL [en ligne] E. aurianticum 16SrRNA, 24 juin 1994 (1994-06-24) retrieved from EBI Database accession no. X70316 XP002247322
- FRUEHLING ANJA ET AL: "Exiguobacterium undae sp. nov. and Exiguobacterium antarcticum sp. nov." INTERNATIONAL JOURNAL OF SYSTEMATIC AND EVOLUTIONARY MICROBIOLOGY, vol. 52, no. 4, juillet 2002 (2002-07), pages 1171-1176, XP009013832 ISSN: 1466-5026 -& DATABASE EMBL [en ligne] Exiguobacterium undae 16S rRNA gene, 21 août 2001 (2001-08-21) retrieved from EBI Database accession no. AJ344151 XP002247323
- DATABASE EMBL [en ligne] Exiguobacterium acetylicum 16S rRNA gene, 10 juillet 1995 (1995-07-10) NAKAGAWA ET AL.: retrieved from EBI Database accession no. D55730 XP002247324

## Description

L'invention a pour objet de nouvelles souches du genre *Exiguobacterium.*

Elle vise également un procédé de culture de ces souches, ainsi que leurs applications industrielles.

L'invention se rapporte plus particulièrement à des souches bactériennes telles qu'isolées d'échantillons provenant de systèmes hydrothermaux marins profonds.

Dans Journal of Clinical Microbiology, vol 38, n°10, October 2000, p.3623-30, Drancourt et al rapportent les résultats de l'étude d'une collection de 177 isolats de sources diverses et de leur identification sur la base des ARNr.

Mais les espèces données de ce tableau sont très éloignées des souches de l'invention.

Dans l'article paru dans International Journal of systematic bacteriology, vol 44, n°1, 1994, p. 74-82, Farrow et al décrivent des études comparatives entre différentes espèces. Le genre *Exiguobacterium* est mentionné, mais aucune précision n'est donnée sur les espèces.

L'article de Frühling et al dans International Journal of systematic and evolutionary microbiology, vol 52, n°4, juillet 2002, p.1171-6, on rapporte des souches de *Exiguobacterium undae sp.nov.* et de *Exiguobacterium antarcticum sp.nov.* isolées à partir de l'eau de mares.

Ces souches et les autres espèces du genre *Exiguobacterium* données ont des positionnements phylogénétiques très éloignés des souches de l'invention.

Le document Data base EMBL, n° d'accession D 55730, donne l'ARNr 16 S d'un clone d'*Exiguobacterium acetylicum,* ce qui correspond aussi à une espèce différente de celle de l'invention.

Ces documents rapportent respectivement, la séquence de l'ARNr 16 S de *Exiguobacterium auriantiacum* (NDCDO 2321) et de *Exiguobacterium undae.* Les commentaires donnés ci-dessus en rapport avec le document Data base s'appliquent également.

L'étude par les inventeurs des échantillons prélevés les a conduit à isoler une nouvelle espèce d'Exiguobacterium présentant des propriétés de grand intérêt dans divers domaines de l'industrie.

L'étude par les inventeurs des échantillons prélevés les a conduit à isoler une nouvelle espèce d'Exiguobacterium présentant des propriétés de grand intérêt dans divers domaines de l'industrie.

L'invention a donc pour but de fournir des souches de cette nouvelle espèce.

Elle vise également à fournir des protocoles de culture de ces souches précisant les conditions physico-chimiques et la composition du milieu de culture qui permettent de produire favorablement des cellules et/ou certains métabolites, plus particulièrement du L(+) lactate.

Selon un autre aspect, l'invention vise l'utilisation directe de ces souches ou celle de leurs métabolites dans divers domaines de l'industrie.

Les souches bactériennes de l'invention sont caractérisées en ce qu'il s'agit *d'Exiguobacterium* de l'espèce *lactigenes* et qu'elles possèdent une séquence d'ADN dont au moins une partie est capable de s'hybrider avec de l'ADN génomique ou plasmidique de la souche déposée le 5 décembre 2002, sous le n° I-2962, à la Collection Nationale de Cultures de Microorganismes (C.N.C.M.), ces souches étant thermotolérantes, saccharolytiques et amylolytiques et/ou capables de produire du L(+)lactate, ayant des propriétés de croissance à des températures de l'ordre de 40 à 50°C, à un pH de 5,4 à 9,15, avec un optimum pour la croissance à 45°C, à un pH de 7 environ, et une teneur de leur ADN en guanine et cytosine de 50 mole% environ, et étant en outre caractérisées par la séquence SEQ ID N°1 de l'ARNr 16S : ou une séquence ayant une similitude avec SEQ ID N°1 supérieure à 97%.

On notera que, de manière avantageuse, le lactate produit par les souches bactériennes de l'invention est à plus de 95 % du L(+) lactate.

L'invention vise plus particulièrement des souches du genre Exiguobacterium tel que montré par comparaison des séquences de l'ARN de la fraction 16 S des ribosomes.

Ces souches sont encore caractérisées par le fait qu'elles ne réduisent pas le sulfate, le thiosulfate, le soufre, le sulfite.

Les souches bactériennes de l'invention sont encore caractérisées en ce qu'elles sont Gram positif.

L'invention vise en particulier la souche bactérienne déposée à la C.N.C.M. le 05 décembre 2002 sous le n° I-2962.

La référence d'identification de cette souche est 10C. Comme nom de désignation taxonomique, on utilisera Exiguobacterium lactigenes sp. nov.

Les mutants des souches répondant aux définitions qui précèdent entrent également dans le cadre de l'invention dès lors qu'ils conservent au moins 70 % de capacité d'hybridation avec l'ADN génomique de la souche déposée.

Conformément à l'invention, les souches bactériennes définies ci-dessus sont obtenues par culture dans des conditions anaérobies facultatives, à un pH de 5,4 à 9,15, à 37°C, dans un milieu de base comme défini ci-après, contenant un sucre utilisable par ces souches comme source d'énergie.

Les souches bactériennes de l'invention sont avantageusement utilisées dans des procédés de fermentation alimentaire. Leurs propriétés fermentaires et enzymatiques permettent d'y remplacer avantageusement et/ou de compléter celles attribuées aux bactéries lactiques utilisées habituellement.

La capacité des souches de l'invention à fermenter une grande variété de sucres, notamment le D-glucose, le D-fructose, le D-galactose, le D-mannose, le mannitol, le D-ribose, le D-saccharose et le DL-maltose et l'amidon constitue un atout important. Certains de ces sucres (glucose, fructose, saccharose) potentiellement utilisables comme substrats énergétiques sont, en effet, disponibles en grande quantité, notamment dans les jus fermentaires sucriers.

La possibilité d'agir sur le métabolisme de ces souches en contrôlant les paramètres physico-chimiques du milieu de culture (pH, rapport sucres/peptides) élargit leur domaine d'application. Ainsi, il est possible par exemple d'orienter la fermentation vers la production de cellules et de métabolites cellulaires tels que des enzymes.

L'invention vise donc également un procédé de production de métabolites, en particulier de L(+) lactate, caractérisé en ce qu'il comprend
- la culture d'une souche bactérienne telle que définie ci-dessus, dans des conditions appropriées pour son développement et pour la production du métabolite recherché,
- la récupération des métabolites produits, suivi de l'isolement du métabolite désiré et sa purification.

L'acide lactique produit par les souches de l'invention est d'un grand intérêt car il est constitué à plus de 95 % par du L(+) lactate qui est assimilable par les organismes supérieurs alors que le D(-) lactate présente un caractère de toxicité.

On le sépare de la culture, et on le concentre par exemple par évaporation, le cas échéant jusqu'à siccité.

Les concentrés ou produits secs sont utilisés tels quels ou traités pour former des dérivés souhaités de l'acide lactique.

Les applications de l'acide lactique ou de ses esters et autres dérivés concernent de nombreux domaines.

L'acide lactique est ainsi utilisé dans l'industrie agro-alimentaire en l'incorporant dans des boissons, des bières, des produits laitiers tels que crème, fromage, beurre, des glaces ou encore des confitures.

Comme tensio-actif, on l'utilisera avec avantage en panification et viennoiserie sous forme par exemple de lactyl mono- et diglycérides et de sodium stéaryl lactylate.

Dans l'industrie pharmaceutique, le lactate de potassium peut constituer un substitut du chlorure de sodium particulièrement précieux dans les cas d'hypertension.

Il est aussi utilisé pour ses propriétés de complexant, notamment avec le fer et le calcium pour traiter les carences.

Enfin parmi les applications de l'acide lactique, de ses sels et dérivés dans l'industrie chimique, on citera son utilisation dans l'élaboration de résines plastiques, d'adhésifs, de pesticides, de textiles, ou encore dans des peintures, des diluants et des solvants, ou pour le traitement de surface de métaux.

On soulignera son grand intérêt dans la chimie des polymères où il sert à fabriquer des polylactides et/ou des copolymères avec par exemple des oxydes de polyalkylène, des alcools polyvalents, de l'acide glycolique, des acides hydroxycarboxyliques, des copolymères d'éthylène et de propylène, des caoutchoucs butyliques ou des élastomères de polyuréthane thermoplastiques. A partir de ces polymères et/ou copolymères, divers articles peuvent être fabriqués en particulier pour l'emballage, des films à usages médicaux pour réaliser des pansements ou encore des matières d'enrobage pour sutures chirurgicales.

D'autres caractéristiques et avantages de l'invention sont rapportés dans la description qui suit, donnée à titre d'exemple, qui concerne la souche 10C mentionnée plus haut, déposée à la C.N.C.M. sous le n°I-2962.

### a. Protocole d'isolement de la souche 10C

L'isolement a été effectué à partir d'échantillons de systèmes hydrothermaux profonds marins.

### . Milieux et méthodes de culture

On utilise un milieu de base contenant (pour 1 litre d'eau distillée) : 1g de NH₄Cl, 0,3g de KH₂PO₄, 0,3g de K₂HPO₄, 25g de NaCl, O, 2g de CaCl₂, 0, 1g de KCl, 3g de MgCl₂, 0, 5g de CH₃COONa, 0, 5g de cystéine-HCl, 0,1g d'extrait de levure (Difco Laboratories), 10ml d'une solution minérale de Balch (1), 1mg de résazurine. Le pH est ajusté à 7,3 avec KOH 10M et le milieu est porté à ébullition sous un courant d'azote et refroidi jusqu'à la température ambiante.

Les compositions de la solution minérale de Balch et de la solution d'oligoéléments de Balch sont les suivantes

| Solution minérale de Balch | |
|---|---|
| KH₂PO₄ | 6 g |
| NH₄)₂SO₄ | 6 g |
| NaCl | 12 g |
| MgSO₄, 7H₂O | 2,6 g |
| CaCl₂, 2H₂O | 0,16 g |
| H₂O distillée q.s.p. | 1000 ml |

| Solution d'oligo-éléments de Balch | |
|---|---|
| Acide nitriloacétique | 1,5 g |
| MnSO₄, 2H₂O | 0,5 g |
| MgSO₄, 7H₂O | 3 g |
| NaCl | 1 g |
| FeSO₄, 7H₂O | 0,1 g |
| CoCl₂, 6H₂O | 0,1 g |
| CaCl₂, 2H₂O | 0,1 g |
| ZnCl₂ | 0,1 g |
| CuSO₄, 5H₂O | 0,01 g |
| AlK(SO₄)₂ | 0,01 g |
| H₃BO₃ | 0,01 g |
| Na₂MoO₄ | 0,01 g |
| H₂O distillée q.s.p. | 1000 ml |

Le pH du milieu de culture est ajusté à pH 7,3 avec KOH 10 M.

Le milieu est ensuite porté à ébullition, puis refroidi jusqu'à la température ambiante et réparti sous un courant d'azote dans des tubes de Hungate, à raison de 5 ml par tube, et dans des flacons de sérum, à raison de 20ml sous courant d'azote et de gaz carbonique (80 :20 ;v/v).

Après traitement à l'autoclave des récipients scellés à 110°C pendant 45 min, on ajoute Na₂S, 9H₂O, Na₂CO₃ et du glucose, à partir de solutions stériles, ce qui conduit, respectivement à des concentrations de 0,04%, 0,2% et 20mM.

Pour initier l'enrichissement de la culture, on inocule un échantillon de 20ml de milieu, et on incube à 37°C. La culture est purifiée en utilisant la méthode des rolls tubes de Hungate avec un milieu solidifié avec 15g/l d'agar.

### b. Description de la souche

La souche 10C est une bactérie à Gram positif, non sporulante, anaérobie facultative , se présentant sous forme de bâtonnets , avec une croissance optimale à 45°C, à pH 7 et 0-2% de NaCl.

Il s'agit d'une souche hétérotrophe qui requiert de l'extrait de levure pour fermenter les sucres.

La température de croissance de la souche est de 12 à 50°C, à un pH de 5,4 à 9,1, et une concentration en NaCl entre 0 et 12%.

On observe une croissance optimale à 45°C, à pH 7 et 0-2% de NaCl.

Dans un milieu contenant des hydrates de carbone, notamment du glucose comme source d'énergie, on ajoutera avec avantage des peptides, par exemple des extraits de levure, pour favoriser la croissance.

### - propriétés métaboliques

La fermentation de sucres conduit essentiellement à du (L+)lactate (environ 2 moles de lactate/mole de glucose fermenté). Dans des conditions de croissance adaptées, on observe la production de formate, acétate et éthanol.

### Caractères génétiques :

La souche 10C est caractérisée par une teneur de l'ADN en guanine + cytosine de 50,4 mole%.

La purification et l'extraction de l'ADN, l'amplification et le séquençage de l'ARNr 16S sont réalisés selon (2), (3) et (4). L'ADN a été isolé par chromatographie sur hydroxyapatite selon le procédé de Cashion et al (5). L'hybridation ADN-ADN a été effectuée comme décrit par De Ley et al (6), avec la modification décrite par Huss et al (7) et Escara et Hutton (8) en utilisant un spectrophotomètre modèle 2600 équipé d'un thermoprogramme 2527-R (Gilford Instrument Laboratories Inc., Oberlin, Ohio, EUA).

La séquence de l'ARNr 16S correspond à SEQ ID N° 1 donnée ci-dessus.

### c. Tableau de différences des substrats entre la souche 10C et E. aurantiacum

| Substrats | 10 C | *Exig aurantiacum* |
|---|---|---|
| Lactate | - | - |
| Benzoate | - | |
| Glucose | + | + |
| Fructose | + | - |
| Galactose | + | + |
| Dx-ylose | - | - |
| Mannose | + | - |
| Mannitol | + | + |
| Gycérol | - | + |
| Fumarate | - | + |
| Pyruvate | - | - |
| Arabinose | - | - |
| Ribose | + | + |
| Sorbose | - | - |
| Sucrose | + | + |
| Maltose | + | + |
| Acétate | - | - |
| Butyrate | - | - |
| Propionate | - | - |
| Casaminoacides | - | - |
| Dulcitol | - | - |
| Lactose | - | - |
| Rhamnose | - | - |
| Melizitose | - | - |

### d. Procédés de culture et applications

### I- PRODUCTION DE BIOMASSE PAR FERMENTATION DE SUCRE

On opère en milieu non renouvelé.

La fermentation est régulée à un pH de 7 à l'aide d'une solution alcaline (soude par exemple) et à une température de 45°C. On utilise un milieu de culture répondant à la composition suivante :
- Glucose à calculer
- Extrait de levure/hydrolysat de protéines à calculer
- NH₄Cl 1 g/l
- NaCl 0,5 g/l
- KH₂PO₄ 0,3 g/l
- K₂HPO₄ 0,3 g/l
- MgCl₂, 6H₂O 0,2 g/l
- KCl 0,1 g/l
- CaCl₂ 2H₂O 0,1g/l

Les concentrations en sucre et en extraits de levure sont fonction de la concentration en cellules que l'on souhaite obtenir.

Le sucre est autoclavé séparément du reste du milieu de culture, ainsi que certains sels minéraux qui forment un précipité lors de l'autoclavage. Ils sont ensuite ajoutés stérilement à l'autre partie du milieu de culture (extrait de levure + minéraux, autoclavés ensemble), puis le volume final est ajusté avec de l'eau distillée stérile. L'exemple ci-dessous permet de mieux comprendre le protocole de préparation des milieux :

### Exemple de préparation de 16 litres d'un milieu à 40 g/l de saccharose et 3 g/l d'extrait de levure :

1°) Pesée et autoclavage

| | Concentration | Masse à peser | |
|---|---|---|---|
| Saccharose | 40 g/l | 640 g | Dans env. 500 ml |
| MgCl₂6H₂O | 0,2 g/l | 3,2 g | d'eau distillée. |
| CaCl₂2H₂O | 0,1 g/l | 1,6 g | Autoclavage 110°C, |
| | | | 20 à 30 min. |
| | | | |
| Extrait de levure | 3 g/l | 48 g | |
| NH4Cl | 1 g/l | 16 g | |
| KH₂PO₄ | 0,3 g/l | 4,8 g | Dans env. 15 1 d'eau |
| K₂HPO₄ | 0,3 g/l | 4,8 g | distillée. |
| NaCl | 0,5 g/l | 8 g | Autoclavage 121°C, 1h30. |
| KCl | 0,1 g/l | 1,6 g | |

| | | | |
|---|---|---|---|
| NB : le sucre est autoclavé dans un faible volume de liquide et seulement 20 minutes à 110°C pour éviter l'hydrolyse du saccharose. Les sels de magnésium et de calcium sont autoclavés à part des autres sels et de l'extrait de levure afin d'éviter toute précipitation. | | | |

2°) Assemblage : la solution à base de sucre est transférée dans les 15 litres de milieu contenant l'extrait de levure, puis de l'eau distillée stérile est ajoutée pour compléter jusqu'à 16 litres. Tous ces transferts se font stérilement, autour de la flamme d'un Bec Bunsen, au moyen d'une surpression d'azote appliquée dans le fût à vider pour pousser le liquide.
3°) Homogénéisation : un flux d'azote N₂ est mis à buller dans le milieu ainsi assemblé afin de mélanger tous les éléments et d'assurer l'anaérobiose.

### 2. Mode de fermentation

Les études ont été réalisées en mode discontinu, ou batch, rendu continu par l'enchaînement des batchs. Il s'agit d'un système de "feed-harvest", ou "batchs répétés", qui se schématise par l'enchaînement séquentiel de trois étapes : remplissage du fermenteur par du milieu neuf, puis culture des bactéries en batch, puis vidange du moût de fermentation en laissant un pied de cuve pour l'inoculation du batch suivant, puis nouveau remplissage, etc.

D'un point de vue pratique, l'avantage de ce système réside dans le fait que les phrases de nettoyage et de stérilisation du fermenteur entre deux batchs sont supprimées, et dans la possibilité d'automatisation du procédé. En effet, il est possible de programmer un automate qui déclenche les opérations de vidange et de remplissage selon la valeur de paramètres acquis en ligne par une unité de régulation.

### II. PRODUCTION DE BIOMASSE PAR FERMENTATION D'AMIDON

Dans d'autres expérimentations, en utilisant un substrat d'amidon et le milieu tamponné défini ci-dessus (mais avec 10 g d'amidon par litre), on obtient une transformation de l'amidon donnant plus de 95% de L(+) lactate et des traces de formiate.

### REFERENCES BIBLIOGRAPHIQUES

(1) Balch W.E. et al, 1979, Microbiol. Rev. 43,260-296,
(2)Andrews K.T. & Patel B.K.C., 1996, Int. J. Syst. Bacteriol. 46, 265-269,
(3) Love C.A. et al, 1993, Syst. Appl. Microbiol. 16, 244-251,
(4)Redburn A.C. & Patel B.K.C., 1993, FEMS Microbiol. Lett. 113, 81-86.
(5) Cashion P., 1977, Anal. Biochem, 81:461-466.
(6) De Ley J, 1970, Eur. J. Biochem, 12:133-142,
(7) Huss V.A.R., 1983, J. Syst. Appl. Microbiol, 4: 184-192,
(8) Escara J.F., 1980, Biopolymers, 19: 1315-1327.

## Revendications

1. Souches bactériennes, **caractérisées en ce qu'**il s'agit *d'Exiguobacterium* de l'espèce *lactigenes* et qu'elles possèdent une séquence d'ADN dont au moins une partie est capable de s'hybrider avec de l'ADN génomique ou plasmidique de la souche déposée le 5 décembre 2002, sous le n° I-2962, à la Collection Nationale de Cultures de Microorganismes (C.N.C.M.), ces souches étant thermotolérantes, saccharolytiques et amylolytiques et/ou capables de produire du L(+)lactate, ayant des propriétés de croissance à des températures de l'ordre de 40 à 50°C, à un pH de 5,4 à 9,15, avec un optimum pour la croissance à 45°C, à un pH de 7 environ, et une teneur de leur ADN en guanine et cytosine de 50 mole% environ, et étant en outre **caractérisées par** la séquence SEQ ID N°1 de l'ARNr 16S : ou une séquence ayant une similitude avec SEQ ID N°1 supérieure à 97%.

2. Souche bactérienne déposée à la C.N.C.M. le 5 décembre 2002, sous le numéro I-2962.

3. Procédé de culture de souches bactériennes selon la revendication 1 ou 2, **caractérisé en ce qu'**on opère dans des conditions anaérobies facultatives, à un pH de 5,4 à 9,15 environ, à 37°C, en particulier de 6,5 à 7,5, dans un milieu de base contenant un sucre utilisable par ces souches comme source d'énergie.

4. Application des souches bactériennes selon la revendication 1 ou 2, dans des procédés de fermentation alimentaire.

5. Procédé de production de métabolites tels que le L(+) lactate, **caractérisé en ce qu'**il comprend
- la culture d'une souche bactérienne selon la revendication 1 ou 2 dans des conditions appropriées pour son développement et pour la production du métabolite recherché,
- la récupération des métabolites produits, l'isolement du métabolite désiré et sa purification.

## Claims

1. Bacterial strains, **characterized in that** they belong to Exiguobacterium of the lactigenes genus and have a DNA sequence, at least part of which is capable of hybridizing with genomic or plasmid DNA of the strain deposited on Dec. 5, 2002, under the No. I-2962, at the Collection Nationale de Cultures de Microorganismes (C.N.C.M.), these strains being thermoresistant, saccharolytic and amylolytic and/or capable of producing L(+) Lactate, having growth properties at temperatures of the order of 40 to 50°C, at a pH of 5.4 to 9.15, with an optimum for growth at 45°C, at a pH of approximately 7, and a guanine plus cytosine content in their DNA of approximately 50 mol %, and further **characterized by** sequence SEQ ID No. 1 of the 16S rRNA: or a sequence having more than 97% similarity with SEQ ID No. 1.

2. The bacterial strain deposited with the C.N.C.M. on Dec. 5, 2002, under the number I-2962.

3. A method for culturing the bacterial strains according to claim 1 or 2, wherein in the process is carried out under facultative anaerobic conditions, at a pH of approximately 5.4 to 9.15, at 37°C., in particular of 6.5 to 7.5, in a basic medium containing a sugar that can be used as an energy source by this strain.

4. The use of the bacterial strains according to claim 1 or 2, in food fermentation processes.

5. A method for producing metabolites such as L(+) lactate, **characterized in that** it comprises:
- culturing a bacterial strain according to claim 1 or 2, under conditions suitable for its development and for the production of the desired metabolite,
- recovering the metabolites produced, isolating the desired metabolite and purifying it.

## Patentansprüche

1. Bakterienstämme, **dadurch gekennzeichnet, dass** es sich um *Exiguobacterium* der Art *lactigenes* handelt und dass sie eine DNA-Sequenz besitzen, von der wenigstens ein Teil in der Lage ist, mit der genomischen oder plasmidischen DNA des Stammes zu hybridisieren, der am 5. Dezember 2002 unter der Nr. 1-2962 bei der Collection Nationale de Cultures de Microorganismes (CNCM) hinterlegt wurde, wobei diese Stämme thermotolerant, saccharolytisch und amylolytisch sind und/oder L-(+)-Lactat produzieren können, Wachstumseigenschaften bei Temperaturen in der Größenordnung von 40 bis 50 °C bei einem pH-Wert von 5,4 bis 9,15 mit einem Wachstumsoptimum bei 45 °C bei einem pH-Wert von ungefähr 7 haben und einen Gehalt ihrer DNA an Guanin und Cytosin von ungefähr 50 Mol-% aufweisen und außerdem durch die folgende Sequenz SEQ ID Nr. 1 der 16S-rRNA: oder eine Sequenz, die eine Homologie mit SEQ ID Nr. 1 von über 97% aufweist, charakterisiert sind.

2. Bakterienstamm, der am 5. Dezember 2002 unter der Nr. 1-2962 bei der CNCM hinterlegt wurde.

3. Verfahren zur Kultivierung von Bakterienstämmen gemäß Anspruch 1 oder 2, **dadurch gekennzeichnet, dass** man unter fakultativ anaeroben Bedingungen bei einem pH-Wert von ungefähr 5,4 bis 9,15 bei 37 °C, insbesondere bei 6,5 bis 7,5, in einem Grundmedium arbeitet, das einen von diesen Stämmen verwertbaren Zucker als Energiequelle enthält.

4. Anwendung von Bakterienstämmen gemäß Anspruch 1 oder 2 in Verfahren zur Nahrungsfermentation.

5. Verfahren zur Produktion von Metaboliten wie L-(+)-Lactat, **dadurch gekennzeichnet, dass** es Folgendes umfasst:
- die Kultivierung eines Bakterienstammes gemäß Anspruch 1 oder 2 unter Bedingungen, die für seine Entwicklung und für die Produktion des gewünschten Metaboliten geeignet sind;
- Gewinnung der produzierten Metaboliten, Isolierung und Reinigung des gewünschten Metaboliten.


### LISTAGE DE SEQUENCES

<110> INSTITUT DE RECHERCHE POUR LE DEVELOPPEMENT (I.R.D.)
<120> SOUCHES BACTERIENNES DU GENRE EXIGUOBACTERIUM PROCEDE DE CULTURE ET APPLICATIONS
<130> CP/VB 60859
<140> 0215865
<141> 2003-12-10
<160> 1
<170> PatentIn version 3.1
<210> 1
   <211> 1510
   <212> DNA
   <213> Exiguobacterium acetylicum
<220>
   <221> misc_feature
   <222> (1117)..(1117)
   <223> unknown
<400> 1
   gcgtgcctaa tacatgcaag tcgagcgcag gaagccgtct gaacccttcg gggggacgac   60
   ggtggaatga gcggcggacg ggtgagtaac acgtaaagaa cctgcccata ggtctgggat  120
   aaccacgaga aatcggggct aataccggat gtgtcatcgg accgcatggt ccgctgatga  180
   aaggcgctcc ggcgtcgccc atggatggct ttgcggtgca ttagctagtt ggtggggtaa  240
   cggcccacca aggcgacgat gcatagccga cctgagaggg tgatcggcca cactgggact  300
   gagacacggc ccagactcct acgggaggca gcagtaggga atcttccaca atggacgaaa  360
   gtctgatgga gcaacgccgc gtgaacgatg aaggctttcg ggtcgtaaag ttctgttgta  420
   agggaagaac aagtgccgca ggcaatggcg gcaccttgac ggtaccttgc gagaaagcca  480
   cggctaacta cgtgccagca gccgcggtaa tacgtaggtg gcaagcgttg tccggaatta  540
   ttgggcgtaa agcgcgcgca ggcggcctct taagtctgat gtgaaagccc ccggctcaac  600
   cggggagggc cattggaaac tgggaggctt gagtatagga gagaagagtg gaattccacg  660
   tgtagcggtg aaatgcgtag agatgtggag gaacaccagt ggcgaaggcg actctttggc  720
   ctataactga cgctgaggct gcgaaagcgt ggggagcaaa caggattaga taccctggta  780
   gtccacgccg taaacgatga gtgctaggtg ttggagggtt tccgcccttc agtgctgaag  840
   ctaacgcatt aagcactccg cctggggagt acggtcgcaa ggctgaaact caaaggaatt  900
   gacggggacc cgcacaagcg gtggagcatg tggtttaatt cgaagcaacg cgaagaacct  960
   taccaactct tgacatcccc ctgaccggta cagagatgta ccttcccctt cgggggcagg 1020
   ggtgacaggt ggtgcatggt tgtcgtcagc tcgtgtcgtg agatgttggg ttaagtcccg 1080
   caacgagcgc aacccttgtc cttagttgcc agcattnagt tgggcactct agggagactg 1140
   ccggtgacaa accggaggaa ggtggggatg acgtcaaatc atcatgcccc ttatgagttg 1200
   ggctacacac gtgctacaat ggacggtaca aagggcagcg aagccgcgag gtggagccaa 1260
   tcccagaaag ccgttctcag ttcggattgc aggctgcaac tcgcctgcat gaagtcggaa 1320
   tcgctagtaa tcgcaggtca gcatactgcg gtgaatacgt tcccgggtct tgtacacacc 1380
   gcccgtcaca ccacgagagt ttgcaacacc cgaagtcggt gaggtaaccg taaggagcca 1440
   gccgccgaag gtggggcaga tgattggggt gaagtcgtaa caaggtagcc gtatcggaag 1500
   gtgcggctga                                                        1510